# EUROPÄISCHE PATENTSCHRIFT

(11) **EP 1 753 379 B1**
(45) Veröffentlichungstag und Bekanntmachung des Hinweises auf die Patenterteilung: **10.03.2010**
(21) Anmeldenummer: 05741838.6
(22) Anmeldetag: 18.05.2005
(51) Int. Cl.: A61F 5/37

(54) **BANDAGE FÜR DEN SCHULTER- UND OBERARMBEREICH**
BANDAGE FOR THE SHOULDER AND THE UPPER ARM
BANDAGE POUR L'EPAULE ET LE BRAS

(30) Priorität: 07.06.2004 DE 102004028604
(43) Veröffentlichungstag der Anmeldung: 21.02.2007
(73) Patentinhaber: Paul Hartmann Aktiengesellschaft, 89522 Heidenheim (DE)
(72) Erfinder: GERMERODT, Hans-Jürgen, 37581 Wanfried (DE)
(74) Vertreter: Langöhrig, Angelika Beate
(86) Internationale Anmeldenummer: PCT/EP2005/005371
(87) Internationale Veröffentlichungsnummer: WO 2005/120404

(56) Entgegenhaltungen:
- EP-A- 0 476 623
- DE-U1- 29 905 488
- US-A- 4 372 301
- US-A- 4 625 719
- US-A- 5 413 552
- US-A- 5 772 617

## Beschreibung

Die Erfindung betrifft eine Bandage für den Schulter- und Oberarmbereich wie sie im medizinischen und orthopädischen Sektor Einsatz findet.

Orthopädische Bandagen üben entsprechend ihrer Konstruktion und ihrem Indikationsfeld eine fixierende, führende, stützende und/oder unterstützende Funktion auf die Extremitäten des menschlichen Körpers aus. Sie müssen dabei den anatomischen Gegebenheiten entsprechend geformt sein, um form- und kraftschlüssig von extern auf den menschlichen Körper einwirken zu können.

Für die Behandlung von Verletzungen des Schulter- und Armbereiches, wie insbesondere Schulterblattbrüchen bzw. -frakturen, Oberarmschaft- und kopffrakturen sowie Oberarmbrüchen, aber auch Verrenkungen und weiteren Verletzungen im Bereich des Oberarms und der Schulter werden sogenannte "Gilchrist-Schulterbandagen" eingesetzt, die eine Ruhigstellung des zu behandelnden Arms am Oberkörper des Patienten ermöglichen.

Im Stand der Technik sind hierzu eine Reihe von Ausgestaltungen derartiger Bandagen bekannt.

So ist beispielsweise aus der EP 0 589 663 A1 eine oberarmfixierende Bandage bekannt, die eine stabile Unterarmschale aufweist sowie eine Oberarmfixierung, wobei die einzelnen Elemente über Führungsschienen miteinander verbunden sind und ein Tragegurt über die Schulter des zu behandelnden Arms geführt ist. Eine derartige Führung ist jedoch nachteilig, da Druck von oben auf den zu behandelnden Arm ausgeübt wird, der sich nachteilig auf die Heilung auswirkt und darüber hinaus auch Schmerzen beim Patienten verursachen kann. Darüber hinaus können durch die Schienen sowie starren Elemente Verletzungen sowie Druckstellen beim Patienten auftreten.

Des Weiteren ist beispielsweise aus der EP 0 911 005 A2 eine Bandage bekannt, die aus einem Oberarmteil sowie einem Unterarmteil gebildet ist, wobei auch hier nachteilig ist, dass das Oberarmteil über die verletzte Schulter läuft und dort einen Druck ausübt. Darüber hinaus ist nachteilig, dass eine Längenverstellbarkeit der Unterarmaufnahme nicht vorgesehen ist, so dass eine entsprechende Bandage in einer Vielzahl von Größen bereitgehalten werden muss.

Aus der WO 98/56322 ist darüber hinaus eine Bandage für den Arm mit Einfassung der Schulter bekannt, wobei hier schalenartige Elemente sowohl zur Aufnahme des Unterarms als auch zur Aufnahme der erkrankten Schulter vorgesehen sind, so dass auch hier auf den zu behandelnden Arm ein negativ zu beurteilender Druck im Schulterbereich ausgeübt wird und darüber hinaus auch hier eine Längenverstellbarkeit der Unterarmschale nicht gegeben ist.

Aus der AT 396 649 B ist ein Bandagensystem bekannt, zur Fixierung von gelenknahen Oberarmfrakturen sowie Verletzungen der Schulter. Die Fixierung erfolgt hier mittels eines Brustgurtes sowie eines Oberarm- und eines Unterarmgurtes, wobei zusätzlich ein Schultergurt angebracht ist. Nachteilig ist hierbei, dass eine Vielzahl von einzelnen Gurten notwendig sind, wobei darüber hinaus durch die Fixierung von Oberarmgurt und Unterarmgurt zum Brustgurt nur eine geringe Anpassbarkeit an verschiedene Patienten gegeben ist.

Des Weiteren offenbart die DE 202 02 102 U1 ein orthopädisches Hilfsmittel wie eine Schulterarmbandage, wobei eine Aufnahme für den verletzten Arm vorgesehen ist sowie eine Schulterkappe, die den Oberarm und die Schulter der verletzten Seite umfasst. Auch hierbei ergibt sich der bereits bekannte Nachteil, dass Druck auf die verletzte Schulter ausgeübt wird.

Eine weitere Schulterarmbandage ist aus der DE 43 06 596 A1 bekannt, die ein einfaches, sicheres und von unnötigen. Schmerzreizen freies Anlegen erlauben soll sowie eine exakte Ruhigstellung bei stabilem Sitz. Es ist hierbei vorgesehen, eine Bandage anzubringen, die den gesamten Oberkörper der Person umschließt und hierbei auch die verletzte Arm- bzw. Schulterseite übergreift. Neben dem bekannten Nachteil weist die Bandage den Nachteil, im Vergleich zu sogenannten "Gurtbandagen", eines erhöhten Wärmestaus auf und nur einer geringen Luftdurchlässigkeit.

Aus der EP 0 736 295 A2 ist ein Schulterfixationsverband bekannt, wobei hier eine Schlauchbandage über den verletzten Arm gezogen wird, wobei auch hier eine Schulterkappe vorgesehen ist, die auf dem verletzten Arm zu liegen kommt. Darüber hinaus ist das Anlegen der Schlauchbandage verhältnismäßig schmerzhaft.

Die US-PS 5,413,552 offenbart eine Schulterarmbandage unter Freilassung der Schulter des verletzten Armes, wobei hier eine Vielzahl von Riemenzügen und Befestigungselementen vorgesehen ist, um eine sichere Fixierung zu erreichen, die ein Anlegen erschweren und darüber hinaus durch die vorgesehenen Schnallen und Schlaufen das Tragen der Bandage unkomfortabel machen.

Das US-Patent 6,485,445 B1 zeigt eine weitere Fixierung einer verletzten Schulter bzw. eines verletzten Arms, wobei hier jedoch keine sichere Fixierung des verletzten Arms am Oberkörper des Patienten erreicht werden kann, da hier die Fixierung des Oberarmes am Oberkörper nicht ausreicht.

Die DE 40 29 662 C1 zeigt eine Fixierbandage und ihre Verwendung, wobei die gezeigte Bandage den Nachteil aufweist, durch die nicht vorgesehene Unterarmführung den verletzten Arm nicht sicher genug zu fixieren.

Schließlich beschreibt die US 4,372,301, die den nächstliegenden Stand der Technik darstellt, eine Fixierbandage mit einer Unterarmaufnahme sowie zwei Gurte, wobei der eine Gurt zum Festlegen der Bandage zwischen Ellbogen und Schulter und der andere Gurt um den Rücken herum verläuft.

Der Erfindung stellt sich daher die Aufgabe, eine Bandage für den Schulter- und Oberarmbereich zu schaffen, die die genannten Nachteile überwindet und insbesondere eine einfach anzulegende Bandage bereitstellt, die im gewissen Rahmen für verschiedene Größen anpassbar ist und eine sichere schmerzarme Fixierung eines verletzten Arms ermöglicht.

Die Erfindung löst diese Aufgabe durch eine Schulter- und Oberarmbereichsbandage mit den Merkmalen des Anspruchs 1. Diese umfasst eine längenverstellbare Unterarmaufnahme zum lösbaren Anbringen am Unterarm eines zu behandelnden Arms eines Patienten sowie zwei daran anschließende Bandabschnitte, wobei der im Bereich eines Ellbogens des Patienten an die Unterarmaufnahme anschließende Bandabschnitt zur Bildung eines Tragegurts von dorsal über die nicht zu behandelnde Schulter nach ventral führbar ist und unter Festlegung einer Winkelung des zu behandelnden Arms fixierbar ist und der an einem Handbereich der Unterarmaufnahme angeordnete Bandabschnitt zur Bildung eines Haltegurts nach dorsal in Richtung zum zu behandelnden Arm verläuft und diesen von posterior nach anterior verlaufend umschließt, wobei der Haltegurt nach der Umschlingung festlegbar ist.

Durch die erfindungsgemäße Gestaltung wird eine Bandage bereitgestellt, die einstückig gestaltbar ist, wobei insbesondere die Unterarmaufnahme aus dem gleichen Material wie die zwei anschließenden Bandabschnitte gefertigt sein kann. Darüber hinaus wird durch die Gestaltung die erkrankte Schulter vollständig freigelassen und durch die längenverstellbare Unterarmaufnahme, die in gewissen Bereichen die Anpassung an verschiedene Patientengrößen zulässt sowie die Einstückigkeit von Halte- und Tragegurt und die unmittelbare Verbindung mit der Unterarmaufnahme eine größtmögliche Flexibilität hinsichtlich des Einsatzes der Bandage erzielt. Durch die Festlegung des Haltegurtes nach Umschlingung des zu behandelnden Armes auf sich selbst z.B. im Bereich des Rückens des zu behandelnden Patienten und/oder auf dem Tragegurt wird eine sichere und stabile Fixierung des Arms am Körper des Patienten erreicht, die eine größtmögliche Ruhigstellung der zu behandelnden Gliedmaße ermöglicht.

Es kann insbesondere vorgesehen sein, dass der zu behandelnde Arm vom Haltegurt zweimal von posterior nach anterior umschlungen wird. Insbesondere erfolgt die Gurtführung bei ein oder zweimaligem Umschlingen von posterior nach anterior nach lateral. Bei der Umschlingung wird der Haltegurt, der im Rückenbereich in Höhe der Lendengegend des Patienten verläuft, zunächst zwischen Oberkörper und zu behandelndem Arm hindurchgeführt und dann über die Vorderseite des Arms um diesen herum nach hinten geschlungen. Dabei kann vorgesehen sein, dass der Haltegurt z.B. nach der ersten Umschlingung des zu behandelnden Arms durch den Haltegurt mit dem Tragegurt festlegbar ist, wobei diese Festlegung insbesondere knapp oberhalb des Ellbogens des Patienten erfolgen kann. Auf diese Weise durch die Festlegung zwischen dem Haltegurt und dem Tragegurt wird eine besonders gute und sichere Fixierung des Arms erzielt, wobei gleichzeitig auf die den Oberarm umschlingende Bandage keine großen Kräfte aufgebracht werden müssen, die eine im schlimmsten Fall Abschnürung des Oberarms zur Folge haben.

Insbesondere kann vorgesehen sein, dass das freie Ende des Haltegurts nach der zweiten Umschlingung wieder in den Rückenbereich des Patienten, also nach dorsal führbar ist und dort auf dem Haltegurt im Bereich der Lendengegend festgelegt wird. Durch die zweimalige Umschlingung, wobei insbesondere der Haltegurt während der zweiten Umschlingung nicht auf der ersten Umschlingung, sondern oberhalb oder unterhalb davon zu liegen kommt, kann eine sichere Stützung und Fixierung des Oberarms erfolgen. Die durch die Fixierung des freien Endes des Haltegurts im Bereich des Rückens aufgebrachten Kräfte finden dabei - unterstützt durch die Festlegung des Haltegurtes mit dem Tragegurtes - ihre Gegenkräfte in der vorderen Fixierung an der Unterarmführung Darüber hinaus erfolgt die erfindungsgemäße Anbringung so schmerzreduziert wie möglich für den Patienten.

Es kann dabei vorgesehen sein, dass der Tragegurt mindestens eine Schlaufe aufweist, durch die der Haltegurt bei und/oder nach der ersten Umschlingung des zu behandelnden Arms führbar ist. Auf diese Weise wird insbesondere eine Fixierung des Haltegurts nach der ersten Umschlingung gegen Verrutschen sichergestellt.

Alternativ und bevorzugt kann jedoch auch ein Velcroelement vorgesehen sein, das neben einer lokalen Fixierung des Haltegurtes auch ein zu festes Zuziehen während der Umschlingung verhindert und eine echte Zwischenbefestigung und damit erhöhte Stabilität gewährleistet. Die Dimension des Velcroelementes kann dabei vorzugsweise so erfolgen, dass der Haltegurt bei der ersten und/oder zweiten Umschlingung des Oberarms mit dem Tragegurt festgelegt wird.

Auch die übrigen Befestigungselemente können Velcroelemente sein, die Haken und Ösenelemente umfassen können. Dabei kann das Ösenelement stets durch die Oberfläche der Bandage selbst gebildet sein. Dafür kann die Bandage als Außenmaterial ein sogenanntes "Flauschmaterial" aufweisen, das den Haken des korrespondierenden Verschlusselementes ausreichend Halt bietet.

Um eine Längenverstellung der Unterarmaufnahme zu ermöglichen, kann vorgesehen sein, dass die Unterarmaufnahme eine Ellbogenführung und/oder -fixierung sowie eine Handführung und/oder -fixierung und schließlich eine Unterarmführung und/oder -fixierung aufweist. Die jeweiligen Fixierungen bzw. Führungen können einstückig an der Unterarmaufnahme angeformt sein. Insbesondere kann die jeweilige Fixierung oder Führung durch jeweils zwei in Querrichtung der Unterarmaufnahme verlaufende Elemente gebildet sein, die aufeinander zur Bildung beispielsweise einer Unterarmfixierung oder -führung fixierbar sind.

Des Weiteren kann vorgesehen sein, dass neben diesen öffen-und schließbaren Führungen bzw. Fixierungen eine Aufnahme für das Ellbogengelenk vorgesehen ist. Insbesondere kann in der Ellbogenführung hierzu eine Öffnung vorgesehen sein, in die das Ellbogengelenk im gekrümmten Zustand aufgenommen und dort gehalten wird. Durch die Verstellbarkeit und Öffenbarkeit der Fixierungen und Führungen können hier verschiedene Weiten und Größen eingestellt werden, so dass die Unterarmaufnahme in gewissem Maße längenverstellbar ist. Dabei kann die Ellbogenfixierung zur Bildung einer Öffnung zur Aufnahme des Ellbogengelenks dienen. Die Unterarmfixierung bzw. -führung kann in etwa auf der Hälfte des Unterarms angeordnet sein, wobei die Handführung und -fixierung so angeordnet ist, dass sie zwischen Daumen und Zeigefinger hindurchgeführt und dort verschlossen wird, so dass eine Verschiebung in Längsrichtung des Unterarms verhindert wird.

Insbesondere kann vorgesehen sein, dass die Bandage aus einem gepolsterten Material bestehen kann, wobei insbesondere ein Lagenmaterial vorgesehen ist, das als Mittelmaterial einen Schaumbereich aufweist, z.B. aus einem Polyesterschaumstoff, der mit einem Flauschmaterial als Außenmaterial zusammenlaminiert ist. Dabei kann das Flauschmaterial insbesondere schweißsaugend sein und z.B. aus Polyamid, Polyester oder Baumwolle sowie Mischungen hiervon bestehen. Darüber hinaus sind sämtliche Materialien möglichst luftdurchlässig ausgeführt, um die Gefahr eines Hitzestaus zu verringern. Im Bereich der Unterarmaufnahme kann dabei vorgesehen sein, als Innenmaterial, das gegen den Körper eines Benutzers anliegt, ebenfalls ein Flauschmaterial vorzusehen, alternativ kann jedoch auch ein anderes hautfreundliches Material dort angeordnet sein. Im Bereich der Bandabschnitte kann insbesondere vorgesehen sein, dass das Material schlauchförmig vernäht sein kann, wobei das Material insbesondere eine Mittelnaht aufweisen kann, zur Verankerung der beiden einander gegenüberliegenden Seiten des zu einem Bandabschnitt abgeflachten Schlauchmaterials aufeinander. Besonders vorteilhaft kann vorgesehen sein, wenn anstelle der Vorsehung von Nähten das Material der Bandage schweißbar ist und so die notwendigen Befestigungen, wie die Anbringung des Halte- sowie des Tragegurts an der Unterarmaufnahme, aber auch entsprechende Abnäher an der Unterarmaufnahme sowie die Besäumung der einzelnen Elemente bzw. die Mittelnaht mittels eines Schweißverfahrens vorgesehen werden können. Als Verfahren können hier bekannte schonende Schweiß- oder Prägeverfahren und auch Ultraschall-Verfahren Anwendung finden. Hierdurch kann die Herstellung erheblich vereinfacht und weniger zeitaufwendig gestaltet werden.

Schließlich kann vorgesehen sein, dass das freie Ende des Tragegurts, nachdem der Tragegurt über die gesunde Schulter des Patienten nach vorne herumgeführt wurde, den Unterarm des Patienten umschlingt, insbesondere zunächst zwischen dem Körper und dem Unterarm des Patienten hindurchgeführt wird und dann auf der gegenüberliegenden Seite des Armes zurückgeführt wird, wobei das freie Ende des Tragegurts dann auf dem Tragegurt selbst festlegbar ist, insbesondere mittels Velcroelementen, so dass auch hier eine stufenlose Verstellbarkeit auch der Winkelung des erkrankten Arms gegeben ist.

Weitere Vorteile und Merkmale der Erfindung ergeben sich aus den übrigen Anmeldeunterlagen und insbesondere aus der nachfolgenden Beschreibung eines Ausführungsbeispiels der Erfindung. Diese soll anhand nachfolgender Zeichnung näher erläutert werden. Dabei zeigen:
Figuren
   1 bis 6 ein Anlegeschema zum Anlegen einer erfindungsgemäßen Bandage.

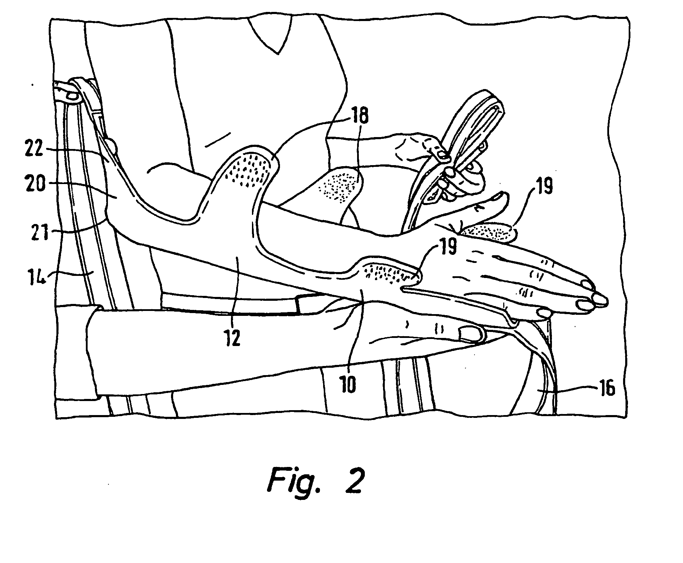

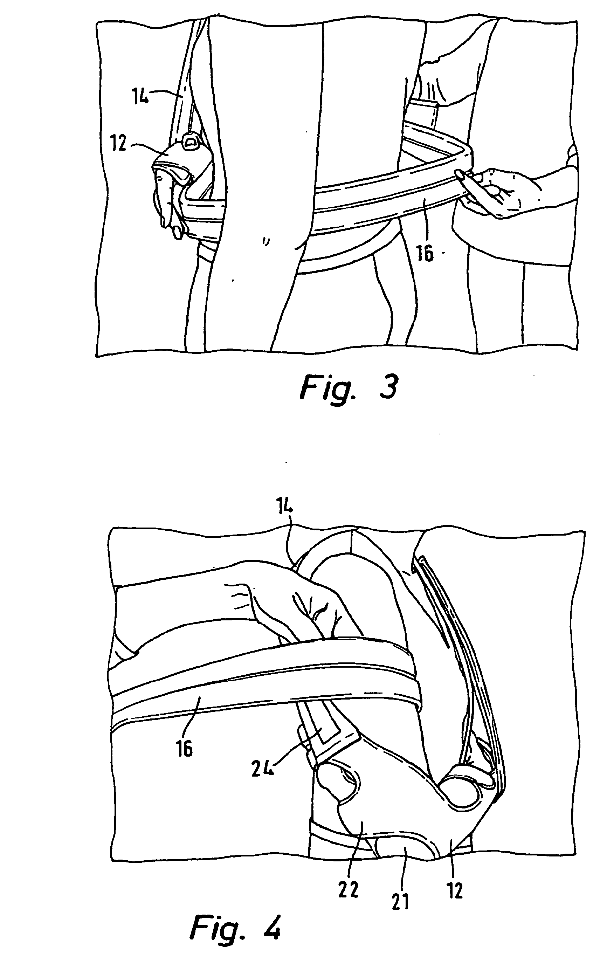

Figur 2 zeigt eine erfindungsgemäße Bandage 10 umfassend eine Unterarmaufnahme 12 sowie zwei Bandabschnitte, von denen einer zur Bildung eines Tragegurts 14 im Ellbogenbereich der Unterarmaufnahme 12 angeordnet ist und der zweite Bandabschnitt zur Bildung eines Haltegurts 16 im Bereich der Handaufnahme der Unterarmaufnahme 12 mit dieser verbunden ist. Die Unterarmaufnahme 12 besteht dabei'aus einem Flachmaterial, das im Bereich der Elle auf den Unterarm auflegbar ist und an der Oberseite des Unterarms mittels einer Unterarmführung und -fixierung 18, die durch zwei Laschen gebildet wird, die aufeinander mittels eines Klettverschlusses festlegbar sind, fixiert wird. Des Weiteren ist eine Handführung und -fixierung 19 vorgesehen, die gleichfalls aus zwei Laschen gebildet ist, die zwischen Daumen und Zeigefinger der Hand des zu behandelnden Arms miteinander verbunden werden und so ein Verrutschen der Unterarmaufnahme 12 in Richtung der Längsachse des Armes verhindern. Des Weiteren ist eine Ellbogenführung und -fixierung 20 vorgesehen, wobei diese zwei Laschen 22 umfasst (Figur 4), die ebenfalls aufeinander fixiert werden können, so dass die Länge der Unterarmaufnahme 12 regulierbar ist und in gewissen Grenzen an die jeweilige Person angepasst werden kann. Des Weiteren weist die Ellbogenführung und - fixierung eine Öffnung 21 auf, die zwischen den zwei Laschen und dem Flachmaterial der Unterarmführung gebildet wird, in der das Ellbogengelenk gelagert werden kann und in der es gegen Verrutschen und Delokalisation der Bandage gesichert ist.

Durch das Zusammenspiel zwischen Handfixierung und Ellbogenfixierung wird zugleich eine größtmögliche Ruhigstellung des Unterarms erreicht, da ein Hin- und Herrutschen des Unterarms in der Unterarmaufnahme 12 verhindert ist.

Nach Fixierung der Unterarmaufnahme 12 am Unterarm des zu behandelnden Arms in beschriebener Weise wird der Tragegurt 14 ausgehend vom Ellbogenbereich des zu behandelnden Arms nach dorsal über den Rücken des Patienten zur nicht zu behandelnden Schulter und über dieselbe geführt, wobei das freie Ende dann den Unterarm, der in der Unterarmaufnahme 12 gehalten ist, umschlingt, wobei die Umschlingung derart erfolgt, dass das freie Ende des Tragegurts 14 zunächst zwischen Patientenkörper und Unterarm durchgeführt und dann wieder auf sich selbst, d. h. dem Tragegurt 14, festgelegt wird (Figur 4). Dabei erfolgt die Fixierung des Armes in einem Winkel von ca. 90° zwischen Oberarm und Unterarm.

Figur 3 zeigt nun die Anbringung des Haltegurts 16, der ausgehend vom Handbereich der Unterarmaufnahme 12 nach dorsal um den Lendenbereich des Patienten herumgeführt wird, um dann zwischen dem verletzten Arm des Patienten und dem Körper des Patienten um den verletzten Arm herumgeführt zu werden, wobei der Haltegurt 16 nach der ersten Umschlingung von posterior nach anterior nach lateral auf einem Velcroelement 24, das oberhalb der Ellbogenfixierung und -führung auf dem Tragegurt 14 angeordnet ist, festgelegt wird (Figur 4).

Hiermit wird eine erste Festlegung des verletzten Arms am Oberkörper des Patienten erreicht.

Figur 4 zeigt darüber hinaus die Ellbogenfixierung und führung mit ihren zwei Laschen 22, die aufeinander mittels eines Velcroelementes festgelegt sind und durch die u. a. eine Verstellung der Länge der Bandage erfolgen kann.

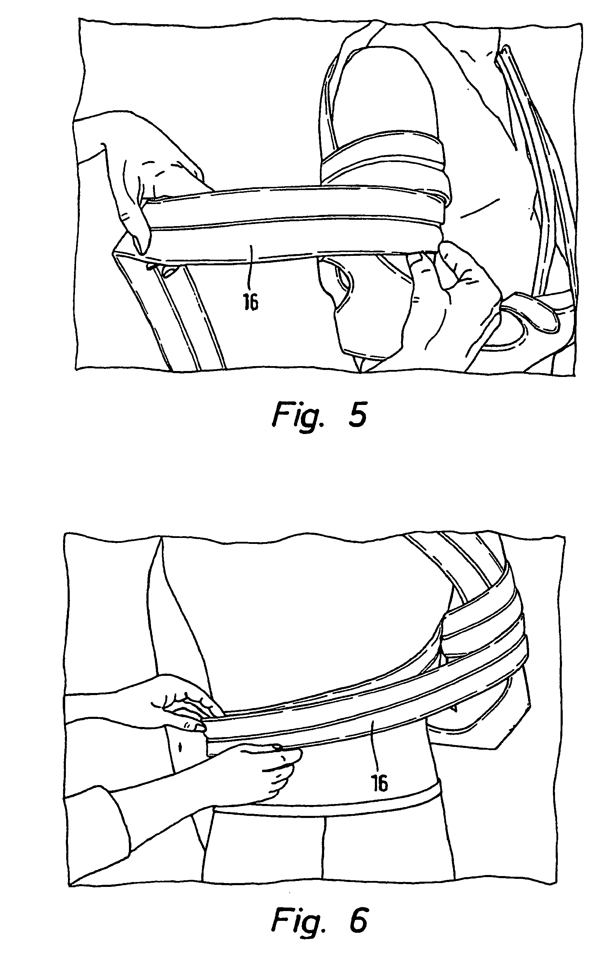

Nach dieser ersten Festlegung erfolgt eine weitere Umschlingung des Oberarms der erkrankten Seite durch den Haltegurt 16, wobei die beiden Umschlingungen des Arms nicht übereinander zu liegen kommen brauchen, sondern nebeneinander in Bezug auf die Längsachse des Oberarms (Figur 5). Nach dieser zweiten Umschlingung erfolgt eine Festlegung des Haltegurtes 16 auf sich selbst im Bereich der Lendengegend des Patienten und ermöglicht eine stufenlose Verstellung (Figur 6). Durch diese Fixierung wird ein sicheres Festlegen des Unterarms und des Oberarms eines Patienten am Körper erreicht, wobei durch die zwei Bandabschnitte, die an gegenüberliegenden Seiten in Längsrichtung der Unterarmaufnahme 12 angeordnet sind, eine gute und stabile Fixierung erreicht werden kann, da die Zugkräfte hier gegeneinander wirken.

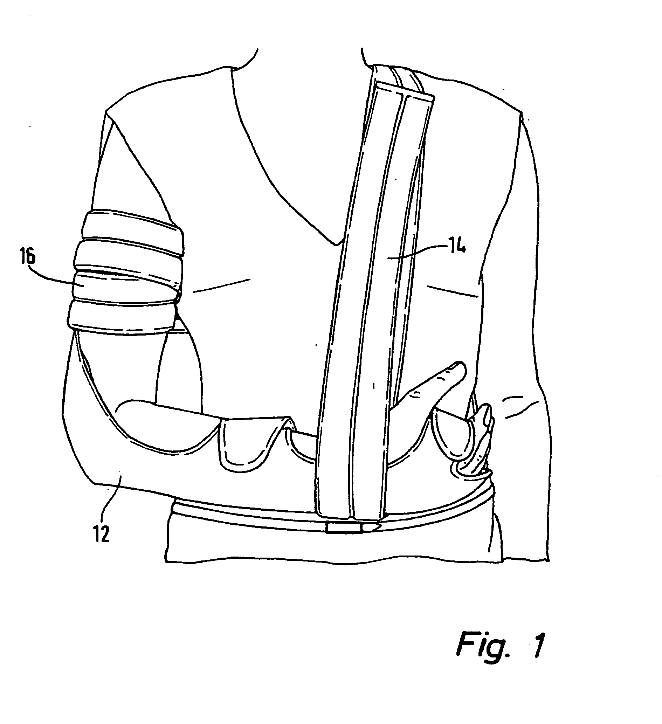

Die gesamte Bandage (Fig 1) besteht hierbei aus einem mehrlagigen Material umfassend ein Polstermaterial aus einem Schaum, der beidseits von einem Flauschmaterial umgeben ist, wobei das Flauschmaterial zugleich als Teil der Klettverschlüsse dient. Darüber hinaus ist ein derartiges Material besonders hautfreundlich und besitzt keine scharfen Kanten, die zu Druck- und Scheuerstellen führen können. Durch die Vorsehung der Klettverschlüsse, wobei ein Teil des Ösenmaterials durch das Flauschmaterial der Bandage selbst gebildet ist, ist eine stufenlose Verstellung sichergestellt. Die vorliegende Bandage liefert daher bei gleichzeitig sicherer Fixierung eine sogenannte "offene" Bandage, die eine größtmögliche Luftdurchlässigkeit und damit Tragekomfort gewährleistet und insbesondere auch hygienische Anforderungen eines sie tragenden Patienten erfüllt.

## Patentansprüche

1. Bandage für den Schulter- und Oberarmbereich umfassend eine längenverstellbare Unterarmaufnahme (12) zum lösbaren Anbringen am Unterarm eines zu behandelnden Arms eines Patienten sowie zwei daran anschließende Bandabschnitte, wobei der im Bereich eines Ellbogens des Patienten an die Unterarmaufnahme (12) anschließende Bandabschnitt zur Bildung eines Tragegurts (14) von dorsal über die nicht zu behandelnde Schulter nach ventral führbar ist und unter Festlegung einer Winkelung des zu behandelnden Arms fixierbar ist und wobei der an einem Handbereich der Unterarmaufnahme (12) angeordnete Bandabschnitt zur Bildung eines Haltegurts (16) nach dorsal in Richtung zum zu behandelnden Arm führbar ist, **dadurch gekennzeichnet, dass** der Haltegurt (16) von posterior nach anterior verlaufend um den zu behandelnden Arm schlingbar ist, wobei der Haltegurt nach der Umschlingung festlegbar ist, dass der Haltegurt (1) bei der Umschlingung des zu behandelnden Arms mit dem Tragegurt (14) im Bereich des Ellbogens festlegbar ist.

2. Bandage nach Anspruch 1, bei der der Haltegurt (16) zweimal von posterior nach anterior um den zu behandelnden Arm schlingbar ist.

3. Bandage nach Anspruch 1 oder 2, bei der das freie Ende des Haltgurts (16) nach dorsal führbar und auf sich selbst festlegbar ist.

4. Bandage nach einem der vorangehenden Ansprüche, bei der auf dem Tragegurt (14) eine Schlaufe vorgesehen ist, durch die der Haltegurt (16) nach der ersten und/oder zweiten Umschlingung des zu behandelnden Arms führbar ist.

5. Bandage nach einem der vorangehenden Ansprüche, bei der die Festlegung über Velcroelemente umfassend Haken- und Ösenelemente erfolgt.

6. Bandage nach Anspruch 5, bei der ein oder mehrere Ösenelemente durch die Oberfläche der Bandage selbst gebildet werden.

7. Bandage nach einem der vorangegangenen Ansprüche, bei der die Unterarmaufnahme (12) eine Ellbogenführung und/oder - fixierung (20) aufweist.

8. Bandage nach einem der vorangegangenen Ansprüche, bei der die Unterarmaufnahme (12) eine Handführung und/oder - fixierung (19) aufweist.

9. Bandage nach einem der vorangegangenen Ansprüche, bei der die Unterarmaufnahme (12) eine Unterarmführung und/oder - fixierung (18) aufweist.

10. Bandage nach einem der vorangegangenen Ansprüche 7 - 9, bei der die Ellbogenführung und/oder -fixierung (20) und/oder die Handführung und/oder -fixierung (19) und/oder die Unterarmfixierung und/oder -führung (18) öffen- und schließbar sind.

11. Bandage nach einem der vorangegangenen Ansprüche, bei der im Bereich der Ellbogenführung und/oder -fixierung (20) eine Öffnung (21) zur Aufnahme des Ellbogengelenks vorgesehen ist.

12. Bandage nach einem der vorangegangenen Ansprüche, bei der das freie Ende des Tragegurts (14) das Handgelenk oder den Unterarm des zu behandelnden Arms umschlingt und dann auf sich selbst festlegbar ist.

13. Bandage nach einem der vorangegangenen Ansprüche, bei der die Bandage (10) aus einem gepolsterten Material besteht.

14. Bandage nach einem der vorangegangenen Ansprüche, bei der das Material der Bandage (10) zumindest zum Teil schweißbar ist.

15. Bandage nach einem der vorangegangenen Ansprüche, bei der die Unterarmaufnahme (12) und die Bandabschnitte aus dem gleichen Material gebildet sind.

## Claims

1. Bandage for the shoulder and upper arm region comprising an adjustable length forearm support (12) for detachable attachment to the forearm of an arm of a patient, which is to be treated, as well as two portions of bandage connected to it, whereby one portion of bandage in the region of the elbow of the patient connected to the forearm support (12) to provide a support strap (14) can be passed from the back to the front over the shoulder that does not require treatment and fixed to establish a required angular setting of the arm requiring treatment and whereby the portion of bandage arranged on a hand region of the forearm support (12) can be passed to the back towards the arm to be treated to provide a retaining strap (16), **characterised in that** the retaining strap (16) can be looped from the back towards the front around the arm that is to be treated, whereby after the looping round the retaining strap can be fixed, such that on looping around the arm that is to be treated the retaining strap (1) can be fixed with the support strap (14) in the region of the elbow.

2. Bandage according to claim 1, in which the retaining strap (16) can be looped twice from the back to the front around the arm that is to be treated.

3. Bandage according to claim 1 or 2, in which the free end of the retaining strap (16) can be passed to the back and fixed to itself.

4. Bandage according to one of the foregoing claims, in which a loop is provided on the support strap (14), through which the retaining strap (16) can be passed after the first and/or second looping around of the arm that is to be treated.

5. Bandage according to one of the foregoing claims, in which the fixing takes place by means of Velcro elements comprising hook and eye elements.

6. Bandage according to claim 5, in which one or more of the eye elements are formed by the surface of the bandage itself.

7. Bandage according to one of the foregoing claims, in which the forearm support (12) has an elbow guide and/or fixing (20).

8. Bandage according to one of the foregoing claims, in which the forearm support (12) has a hand guide and/or fixing (19).

9. Bandage according to one of the foregoing claims, in which the forearm support (12) has a forearm guide and/or fixing (18).

10. Bandage according to one of the foregoing claims 7 - 9, in which the elbow guide and/or fixing (20) and/or the hand guide and/or fixing (19) and/or the forearm guide and/or fixing (18) can be opened and closed.

11. Bandage according to one of the foregoing claims, in which an opening (21) is provided in the region of the elbow guide and/or fixing (20) for the acceptance of the elbow joint.

12. Bandage according to one of the foregoing claims, in which the free end of the support strap (14) is looped around the wrist or the forearm of the arm that is to be treated and can then be fixed to itself.

13. Bandage according to one of the foregoing claims, in which the bandage (10) consists of a padded material.

14. Bandage according to one of the foregoing claims, in which the material of the bandage (10) is at least partly weldable.

15. Bandage according to one of the foregoing claims, in which the forearm support (12) and the portion of bandage are formed from the same material.

## Revendications

1. Bandage pour la zone de l'épaule et de l'humérus comprenant un logement pour l'avant-bras (12) réglable en longueur destiné à venir soutenir de façon amovible l'avant-bras d'un bras à soigner, ainsi que deux segments de bande venant s'y raccorder, le segment de bande venant se raccorder au logement pour l'avant-bras (12) dans la zone du coude du patient en vue de former une sangle (14) pouvant être guidé de l'arrière vers l'avant par l'intermédiaire de l'épaule non endommagée et peut être fixé après détermination de l'inclinaison du bras à soigner, le segment de bande agencé au niveau de la zone de la main du logement pour l'avant-bras (12) en vue de former une ceinture de maintien (16) pouvant être guidé vers l'arrière en direction du bras à soigner, **caractérisé en ce que** la ceinture de maintien (16) peut être enroulée autour du bras à soigner de l'arrière vers l'avant, la ceinture de maintien pouvant être fixée à la suite de cet enroulement, et **en ce que** la ceinture de maintien (16) peut être fixée avec la sangle (14) dans la zone du coude lors de l'enroulement autour du bras à soigner.

2. Bandage selon la revendication 1, **caractérisé en ce que** la ceinture de maintien (16) peut être enroulée deux fois d'arrière en avant autour du bras à soigner.

3. Bandage selon la revendication 1 ou 2, **caractérisé en ce que** l'extrémité libre de la ceinture de maintien (16) peut être guidée vers le dos et peut se fixer sur elle-même.

4. Bandage selon l'une des revendications précédentes, **caractérisé en ce qu'**il est prévu sur la sangle (14) une boucle à travers laquelle la ceinture de maintien (16) peut être guidée après le premier et/ou le deuxième enroulement(s) autour du bras à soigner.

5. Bandage selon l'une des revendications précédentes, **caractérisé en ce que** la fixation se fait au moyen d'éléments du type velcro® comprenant des éléments de type crochets et des éléments de type boucles.

6. Bandage selon la revendication 5, **caractérisé en ce qu'**un ou plusieurs éléments de type boucles sont formés par la surface du bandage.

7. Bandage selon l'une des revendications précédentes, **caractérisé en ce que** le logement pour l'avant-bras (12) présente un guidage et/ou une fixation pour le coude (20)

8. Bandage selon l'une des revendications précédentes, **caractérisé en ce que** le logement pour l'avant-bras (12) présente un guidage et/ou une fixation pour la main (19).

9. Bandage selon l'une des revendications précédentes, **caractérisé en ce que** le logement pour l'avant-bras (12) présente un guidage et/ou une fixation pour l'avant-bras (18).

10. Bandage selon l'une des revendications précédentes 7 à 9, **caractérisé en ce que** le guidage et/ou la fixation du coude (20) et/ou le guidage et/ou la fixation de la main (19) et/ou le guidage et/ou la fixation de l'avant-bras (18) s'ouvrent et se ferment.

11. Bandage selon l'une des revendications précédentes, **caractérisé en ce qu'**il est prévu dans la zone du guidage et/ou de la fixation du coude (20) une ouverture (21) destinée à recevoir l'articulation du coude.

12. Bandage selon l'une des revendications précédentes, **caractérisé en ce que** l'extrémité libre de la sangle (14) passe autour du poignet ou de l'avant-bras du bras à soigner puis vient se fixer sur elle-même.

13. Bandage selon l'une des revendications précédentes, **caractérisé en ce que** le bandage (10) est composé d'un matériau capitonné.

14. Bandage selon l'une des revendications précédentes, **caractérisé en ce que** le matériau du bandage (10) est au moins en partie soudable.

15. Bandage selon l'une des revendications précédentes, **caractérisé en ce que** le logement pour l'avant-bras (12) et les segments de bande sont formés dans le même matériau.
